(19) Europäisches Patentamt

European Patent Office

Office européen des brevets

(11) **EP 1 192 443 B1**

(12) **FASCICULE DE BREVET EUROPEEN**

(45) Date de publication et mention
de la délivrance du brevet:
**02.05.2003 Bulletin 2003/18**

(21) Numéro de dépôt: **00948067.4**

(22) Date de dépôt: **30.06.2000**

(51) Int Cl.$^7$: **G01N 15/14**, G01N 33/569

(86) Numéro de dépôt international:
**PCT/FR00/01868**

(87) Numéro de publication internationale:
**WO 01/002835 (11.01.2001 Gazette 2001/02)**

(54) **PROCEDE DE QUANTIFICATION DE MICROPARTICULES ENDOTHELIALES**

QUANTIFIZIERUNGSVERFAHREN FÜR ENDOTHELIALE MIKROTEILCHEN

METHOD FOR QUANTITATION OF ENDOTHELIAL MICROPARTICLES

(84) Etats contractants désignés:
**AT BE CH CY DE DK ES FI FR GB GR IE IT LI LU
MC NL PT SE**

(30) Priorité: **01.07.1999 FR 9908505**

(43) Date de publication de la demande:
**03.04.2002 Bulletin 2002/14**

(73) Titulaire: **Biocytex
13010 Marseille (FR)**

(72) Inventeurs:
• **CANTON, Michel
F-92300 Levallois-Perret (FR)**
• **DIGNAT-GEORGE, Françoise
F-13007 Marseille (FR)**

(74) Mandataire: **Warcoin, Jacques et al
Cabinet Régimbeau,
20, rue de Chazelles
75847 Paris Cedex 17 (FR)**

(56) Documents cités:
• **V. COMBES ET AL: "Endothelial
microparticules: induction by TNF in vitro and
possible implications for dissemination for
prothrombibotic activity in patients with lupus
anticoagulant" THE JOURNAL OF CLINICAL
INVESTIGATION, vol. 104, no. 1, 1 juillet 1999
(1999-07-01), XP000913435 cité dans la demande**
• **V. LATGER ET AL.: "Vascular disease and cell
activation: exploration of cell adhesion
phenotype by quantitative flow cytometry"
JOURNAL DES MALADIS VASCULAIRES, vol.
24, no. 1, février 1999 (1999-02), pages 11-18,
XP000910966 ISSN 0398-0499**
• **M. MUTIN ET AL.: "Immunologic phenotype of
cultured endothelial cells: quantitative analysis
of cell surface molecules" TISSUE ANTIGENS,
vol. 50, no. 5, novembre 1997 (1997-11), pages
449-458, XP000911054 ISSN 0001-2815**
• **P. PONCELET ET AL.: "Quantitation of
Hematopoietic-cell antigens in flow cytometry"
EUROPEAN JOURNAL OF HISTOCHEMISTRY,
vol. 40, no. 1, 1996, pages 15-32, XP000911015
ISSN: 1121-760X**
• **JW GRAMATAMA ET AL.: "Flow cytometric
quantitation of immunoflurescence intensity:
Problems and perspectives" CYTOMETRY, vol.
33, no. 2, 1 octobre 1998 (1998-10-01), pages
166-178, XP000910968 issn 0196-4763**

## Description

La présente invention concerne un monoréactif et une trousse de diagnostic le comprenant destinés à la détection et à la quantification des microparticules d'origine endothéliale dans un échantillon plasmatique par cytométrie de flux. Le monoréactif comporte un mélange d'anticorps monoclonaux spécifiques des molécules membranaires des cellules endothéliales, ledit mélange comprenant un anticorps (1) marqué avec un fluorochrome 1 et un anticorps (2) marqué avec un fluorochrome 2, lesdits anticorps (1) et (2) n'ayant pas la même spécificité et quatre populations de microsphères servant respectivement à définir la région d'analyse, le seuil d'intensité minimum et permettant le comptage des microparticules. L'invention porte également sur un procédé de quantification des microparticules d'origine endothéliale mettant en oeuvre le monoréactif et la trousse.

Sous l'action de certains stimuli, tels que des cytokines, des interleukines ou des agents infectieux, la membrane plasmique des cellules endothéliales produit des microvésicules. Ce phénomène de bourgeonnement de la membrane pourrait exposer une plus grande surface catalytique pour l'assemblage du complexe enzymatique prothrombinasique qui est une étape essentielle de la cascade de la coagulation génératrice de thrombine, l'enzyme clé de la fibrinoformation. L'existence de microvésicules d'origine endothéliale, et donc de marqueurs d'une activation ou d'une souffrance endothéliale, pourrait être une cause ou une conséquence de lésions vasculaires aiguës ou chroniques.

Une étude, à la base de laquelle la présente invention a été accomplie, a démontré que des microparticules endothéliales (EMP) sont présentes dans le sang périphérique humain (Combes V. et al. 1er juillet 1999. Endothelial microparticles : induction by TNF in vitro and possible implications for dissemination for prothrombotic activity in patients with lupus anticoagulant ; *The Journal of Clinical Investigation* ; vol. 104, n° 1, incorporé dans la description par référence).

Pour la première fois en effet, des EMP ont été détectées en forte proportion dans le plasma de patients présentant un lupus anticoagulant (LA). Chez ces patients, la coagulation anormale a été reconnue comme étant fortement associée aux risques de thromboses (Harris E., Pierangeli S., Gharavi. A. E. 1998. Diagnosis of the antiphospholipid syndrome: a proposai for use of laboratory tests. *Lupus 7* : S144-S148 ; et Whal, D. G., Guillemin F., de Maistre E., Perret C., Lecompte T. and Thibaut G. 1997. Risk for venous thrombosis related to antiphospholipid antibodies in systemic lupus erythematosus - A meto-analysis. *Lupus 6*: 467-473 ; incorporé dans la description par référence).

Dans Combes et al., 1999, la quantification des EMP a été effectuée par cytométrie de flux comme suit: Des suspensions contenant $10^5$ cellules ou des microparticules produites par $10^6$ cellules ont été analysées par Coulter™ Epix™ XL (Coultronics France, Margency). Les microparticules ont été définies comme étant des éléments de diamètre inférieur à 1,5 µm et marqués avec des anticorps spécifiques. Le nombre de particules est évalué en utilisant des anticorps marqués avec FITC-annexine V, comme précédemment décrit dans Jy, W., Horstman L. L, Arce M., and Ahn U. S.. 1992. Clinical significance of platelet microparticles in autoimune thrombocytopenias. *J. Lab. Clin. Med.* 119 : 334-345, incoporé dans la description par référence. Des billes de latex calibrées (Sigma) ont été utilisées comme standard et un nombre connu de billes a été ajouté à chaque échantillon.

La publication Combes et al montre qu'il existe dans le plasma de patients présentant les symptômes de la thrombose un nombre significativement plus important d'EMP que dans le plasma de patients asymptômatiques.

La technique de cytométrie de flux est bien connue de l'homme du métier et est couramment utilisée pour la quantification et la discrimination des cellules et des microvésicules. On peut citer par exemple la méthode visant à quantifier les microvésicules dérivées des plaquettes dans le plasma telle que décrite dans Combes et al, 1997. A new flow cytometry method of platelet-derived microvesicule quantitation in plasma. *Thrombosis and Haemostasis* 77 (1) page 8. Toutefois, cette publication ne décrit pas un procédé de détection et de quantification de microparticules endothéliales consistant en un double marquage et mettant en oeuvre la population distincte de microsphères.

V. Latger et al., Journal des maladis vasculaire, vol.24, no.1, février 1999 (1999-02), pages 11-18 décrit un monoréactif qui comprend un mélange d'anticorps monoclonaux spécifiques des molécules membranaires des cellules endothéliales, lesdits anticorps n'ayant pas la même spécificité et un mélange de microsphères, comprenant:

- une population A de microsphères marquée par un fluorochrome
- une population de microsphères B dont le nombre est connu.

## Description

La présente invention concerne un perfectionnement et une adaptation de la technique utilisée dans Combes et al pour quantifier les EMP dans le plasma. Le procédé, objet de l'invention, consiste en un double marquage qui permet non seulement d'amplifier le signal mais également de calculer le ratio entre deux marqueurs et ainsi, de suivre l'état physiologique des patients.

Un premier aspect de l'invention porte sur un monoréactif pour la détection et la quantification des microparticules d'origine endothéliale dans un échantillon plasmatique par cytométrie de flux caractérisé en ce qu'il comprend un mélange d'anticorps monoclonaux spécifiques des molécules membranaires des cellules endothéliales, ledit mélange comprenant un anticorps (1) marqué avec un fluorochrome 1 et un anticorps (2) marqué avec un fluorochrome 2, lesdits anticorps (1) et (2) n'ayant pas la même spécificité et un mélange de microsphères comprenant :

- Une population A de microsphères (étalon) marquée par un fluorochrome servant à définir la région d'analyse des microparticules en terme de taille (paramètres de diffusion lumineuse) et permettant leur différenciation,
- une population de microsphères B (étalon) dont le nombre est connu servant au comptage des microparticules,
- une population de microsphères C marquée uniquement par le fluorochrome 1, servant à définir sur le paramètre de fluorescence du fluorochrome 1 le seuil d'intensité minimum caractérisant les microparticules marquées par les anticorps-fluorochrome 1,
- et une population de microsphères D, de diamètre identique à C, et marquée uniquement par le fluorochrome 2, servant à définir la région d'analyse des événements considérés positifs pour le fluorochrome 2.

Un tel monoréactif permet le marquage en double couleur des microparticules endothéliales de telle sorte que la différenciation entre lesdites microparticules et leur équivalent, notamment leur équivalent plaquettaire, est optimisée. Il permet également la standardisation des seuils de discrimination des microparticules endothéliales des autres particules et débris susceptibles d'êtres responsables d'un bruit de fond, et la standardisation du comptage des microparticules endothéliales par rapport aux microsphères étalons mentionnées ci-dessus.

Avantageusement le monoréactif comprend un mélange d'anticorps monoclonaux sélectionnés notamment parmi CD51-PE (marqueur de la vitronectine, désigné également par alpha V beta 3, clone AMF7, Immunotech, Marseille, France), CD9-PE (Clone SN4/C3-3A2 Alexis Corporation), CD34-PE (clone 43.A1, Alexis Corporation), CD146-FITC (clone S-Endo 1, COM2F6 ou COM3D9, Biocytex). Bien entendu, tout anticorps équivalent provenant d'une autre source peut être utilisé pour mettre en oeuvre l'invention. De préférence, les anticorps anti-CD51 et anti-CD146 sont utilisés.

Dans un mode avantageux d'exécution, les microsphères A ont un diamètre compris entre 0,5 et 1 μm, préférentiellement 0,8 μm, et les microsphères B et C ont un diamètre compris entre 2 et 10 μm, préférentiellement 3 μm. Le diamètre des microsphères C et D peut être différent de celui des billes B de manière à dissocier de façon simple par les paramètres de diffusion lumineuse les populations C + D de la population B. Le diamètre des microsphères B, C et D peut aussi être identique. Les populations C et D peuvent être présentes dans une proportion relative de 1/3 à 2/3, préférentiellement 1/2.

Les matériaux suivants peuvent être utilisés comme support solide :

Les polymères organiques, tels que les polysaccharides, les polymères de styrène, les polyacrylates, la polyacrylamide, l'hydroxyethyl polyméthacrylates, les polyvinyls, les polystyrènes et les polymères contenant des groupes aromatiques. Le support solide préféré est à base de latex.

De préférence, les fluorochromes sont choisis notamment parmi la phycoérythrine et le FITC (fluresceine isothiocyanate). Néanmoins, on peut également citer le duochrome, l'allophycocyanine, le PE Tandem, l'ultralite 700 et le Texas red (ces flurochromes sont disponibles dans le commerce).

Un deuxième aspect de l'invention a pour objet une trousse de diagnostic comprenant un monoréactif tel que mentionné ci-dessus destinée à la détection et à la quantification de microparticules endothéliales dans le plasma humain.

Cette trousse de diagnostic peut comprendre en outre un échantillon contrôle négatif de référence provenant d'un pool de plasmas humains normaux déplétés en plaquettes, ledit échantillon étant sous la forme liquide ou lyophilisé.

Un aspect complémentaire de l'invention a trait à un procédé de quantification des microparticules endothéliales dans le sang caractérisé en ce qu'il comprend les étapes suivantes :

a) Préparation de plasma déplété en plaquettes par centrifugation et microfiltration,
b) Incubation du plasma avec un monoréactif selon l'invention de sorte à obtenir un marquage en double couleur desdites microparticules,
c) Discrimination et comptage des microparticules par cytométrie de flux.

Les articles et brevets suivants reprennent l'ensemble des données concernant la technique de l'analyse par cytométrie de flux et sont incorporés dans la description par référence :

- McHugh, T. M., "Flow Cytometry and the Application of Microsphere-Based Fluorescence Immunoassays", *Immunochemica*, vol. 5 (1991).
- Shapiro, H. M., *Practical Flow Cytometry*, Ch. 7, pp. 115-198 (1988).
- Gosling, "A Decade of Development in Innunoassay Methodology", *Clin. Chem.*, vol. 36, pp. 1408-1427 (1990).

- Sigma, Immunochemicals Catalog, p. 285 (1992).
- Cappel Product Guide (1991).
- Bangs, "Latex Agglutination Tests", *Am. Clinical Lab. News Edition* (1988).
- Cantarero et al., "The Adsorptive Characteristics of Proteins for Polystyrene and Their Significance in solid-Phase Immunoassays", *Analytical Biochemistry*, vol. 105, pp. 375-382 (1980).
- Wilson et al., "A New Microsphere-based Immunofluorescence Assay using Flow Cytometry", *J. Immuno. Meth.*, vol. 107, pp. 225-230 (1988).
- Bangs, "Microsphere-Based Tests and Immunoassays for Fun and Profit", Bangs *Laboratories Seminar* (1991).
- Rhone-Poulenc, "Estapor-brand Microspheres", Bangs Laboratories Technical Reference #21.
- Walker et al., (Eds), *Techniques in Molecular Biology*, pp. 113-135 and 273-283 (1982).
- Fulwyler et al., "Flow Microsphere Immunoassay for the Quantitative and Simultaneous Detection of Multiple Soluble Analytes", *Methods in Cell Biology*, vol. 33, pp. 613-619 (1990).
- US 4,499,052 ; US 4,526,276 ; US 4,717,655 ; US 4,859,584 ; US 4,783,401 et US 4,762,701, WO 98/21593 et US 5,567,627 en particulier de la page 8 à la page 14.

Le plasma déplété en plaquettes peut être obtenu par double centrifugation et filtration sur Millipore 1μm ou par toute méthode équivalente. De préférence, on effectue un première centrifugation pendant 10 à 15 min, entre 2 et 18°C et à 1500 G environ, on reprend le surnageant décanté, puis on réalise une deuxième centrifugation dans les mêmes conditions.

Un aspect supplémentaire de l'invention porte sur l'utilisation d'un monoréactif ou d'une trousse explicité ci-dessus pour la détection des activités procoagulantes au cours des maladies thrombotiques ou prothrombotiques telles que les lupus anti-coagulants et/ou pour la détection de lésions vasculaires aiguës ou chroniques.

**Exemples 1 : Caractéristiques préférées des microsphères.**

Le monoréactif comporte, en plus des anticorps marqués, pour les uns par un fluorochrome 1 (ex. FITC) et pour les autres par fluorochrome 2 (ex. phycoérythrine), 4 populations de microsphères.
Ces 4 populations de microsphères jouent dans l'analyse cytométrique de l'échantillon les rôles spécifiques suivants :

a) Une population de microsphères A (μS A), de diamètre compris entre 0,5 et 1 μm, préférentiellement 0,8 μm, sert à définir la région d'analyse des microparticules en terme de taille (paramètres de diffusion lumineuse/ « scatters »). Préférentiellement, ces microsphères A sont porteuses d'un fluorochrome permettant leur différenciation simple au sein de l'échantillon biologique.

b) Une population de microsphères B (μS B), de diamètre compris entre 2 et 10 μm, préférentiellement 3 μm, et dont le nombre absolu dans le test est parfaitement connu, sert au comptage absolu des microparticules contenues dans l'échantillon.

c) Une population de microsphères C (μS C), de diamètre compris entre 2 et 10 μm et marquée uniquement par le fluorochrome 1, sert à définir sur le paramètre de fluorescence 1 le seuil d'intensité minimum caractérisant les microparticules marquées par les anticorps- fluorochrome 1.

d) Une population de microsphères D (μS D), de diamètre identique à C et marquée uniquement par le fluorochrome 2, sert à définir la région d'analyse des événements considérés positifs pour le fluorochrome 2.

Préférentiellement, le diamètre des microsphères C et D est choisi différent de celui des billes B pour dissocier de façon simple par les scatters les populations C + D de la population B (par ex. C et D = 5 μm et B = 3 μm). Mais on peut aussi préférer associer B, C et D à une même taille (par exemple B = C = D = 3 μm) et utiliser l'ensemble (B + C + D), homogène en taille, pour les fonctions de comptage et de réglage des fluorescences.

Les populations C et D sont représentées dans le mélange dans une proportion relative de 1/3 à 2/3, préférentiellement 1/2. Les caractéristiques de fluorescence des microsphères C et D sont les suivantes :

L'intensité de la fluorescence moyenne est comprise entre 3 et 100 fois, préférentiellement 10 à 30 fois l'intensité des microparticules d'un échantillon contrôle négatif traité selon l'invention. L'échantillon contrôle négatif de référence est réalisé à partir d'un pool de plasma humaine normaux, pauvres en plaquettes, filtré sur filtre de 1 μm, congelé en aliquots. Un aliquot est marqué avec les monoréactifs et dans les conditions du test, en présence d'un excès 10 x molaire de chacun des anticorps constituant les monoréactifs (inhibition du marquage fluorescent).

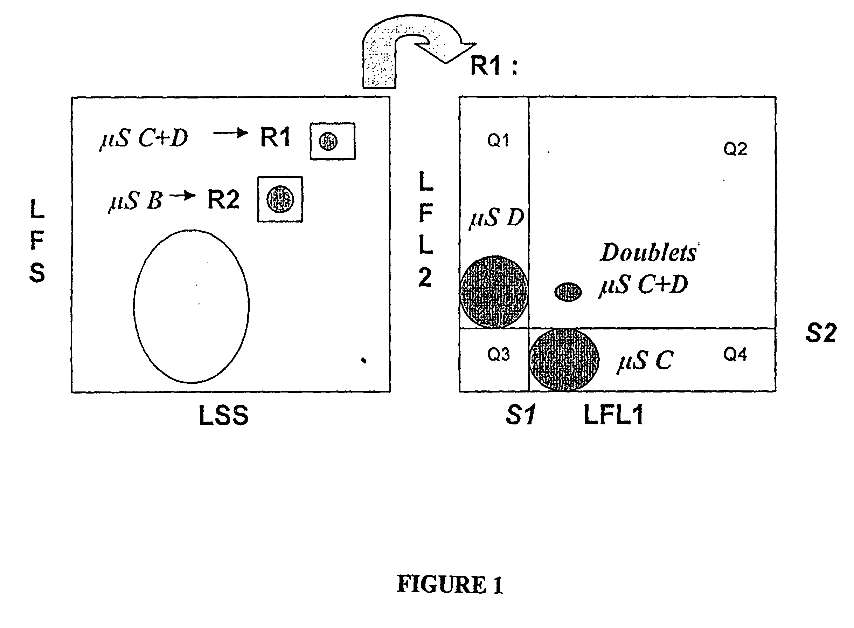

L'intensité de fluorescence moyenne des microsphères C (resp. D) est prise comme référence stable pour établir la position du seuil de positivité des microparticules pour le fluorochrome 1. De même, les microsphères D sont prises comme référence stable pour établir la position du seuil de positivité des microparticules pour le fluorochrome 2. Ces seuils sont définis, lot par lot, pour qu'une majorité (70 % à 99,999 %, préférentiellement 98 %) des microparticules de l'échantillon contrôle négatif de référence, défini ci-dessus, soit située dans la région d'intensité de fluorescences inférieur aux valeurs seuils (figure 1, région Q3). Le coefficient multiplicateur (alpha) reliant l'intensité de fluorescence moyenne de la bille (référence stable) et la valeur d'intensité de fluorescence du seuil correspondant est un paramètre caractéristique de lot. Il permet de positionner chaque seuil de façon reproductible selon la formule :

Intensité de fluol du seuil = alpha x MFI µS C.

**Exemple 2 : Illustration du procédé selon l'invention en trois étapes**

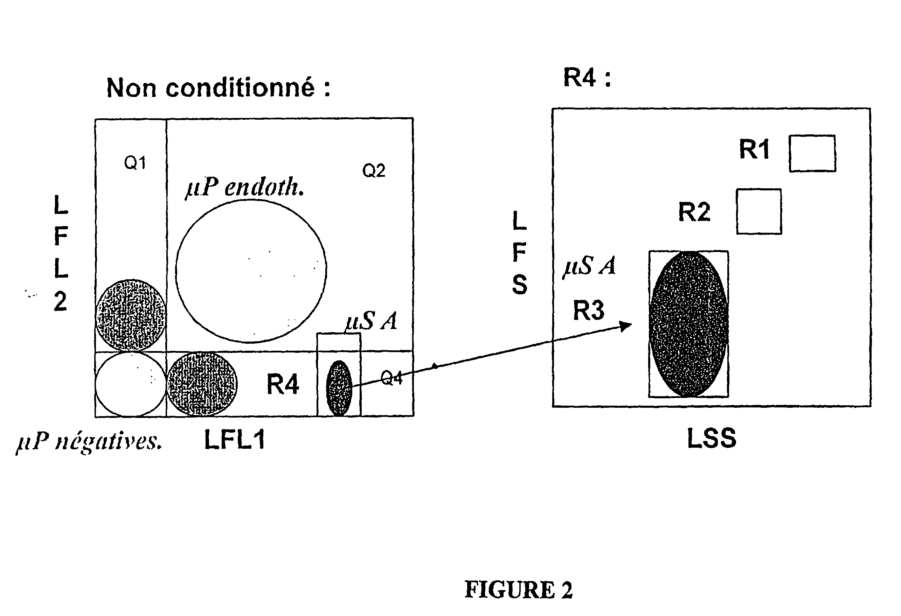

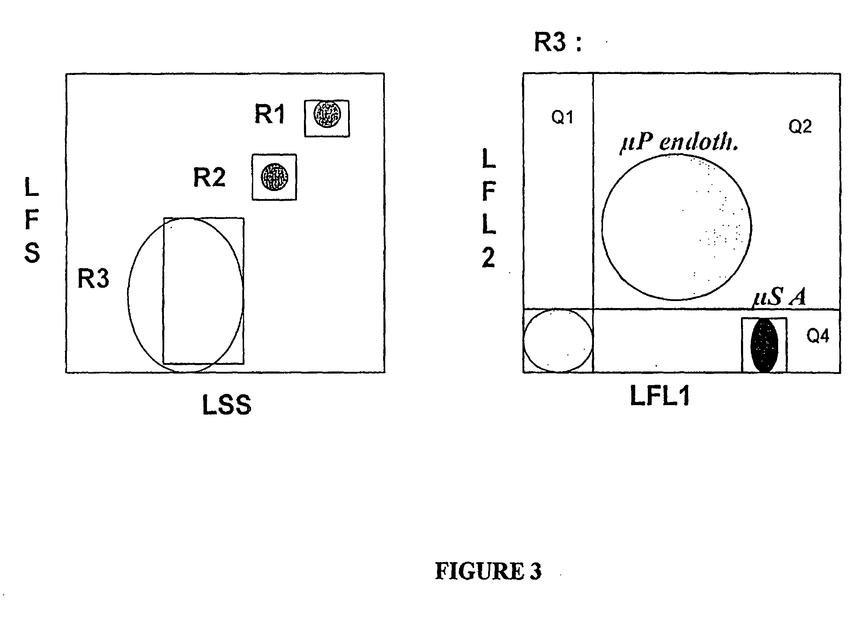

Etape 1 : Cette étape consiste à sélectionner les microsphères C et D (µS C+D) correspondant aux valeurs de fluorescence dans la région R1 de la figure 1. Les paramètres de fluorescence (PMTv, compensations) et les niveaux seuils (figure 1, S1 et S2) sont réglés.
Etape 2 : Les fluorescences sont analysées sans conditionnement. Les microsphères A sont sélectionnées sur LFL1 (figure 2, région R4). La région R3 définissant les microparticules endothéliales (µP endoth) qui contient les microsphères A (µS A) est réglée.
Etape 3 : Les échantillons sont analysés en sélectionnant les microparticules dans la région R3 (figure 3). Un nombre donné de microsphères B (µS B) (région R2) est déterminé et on compte les microparticules (région Q2).

**Exemple 3 : Quantification des EMP chez un patient atteint d'un lupus anticoagulant**

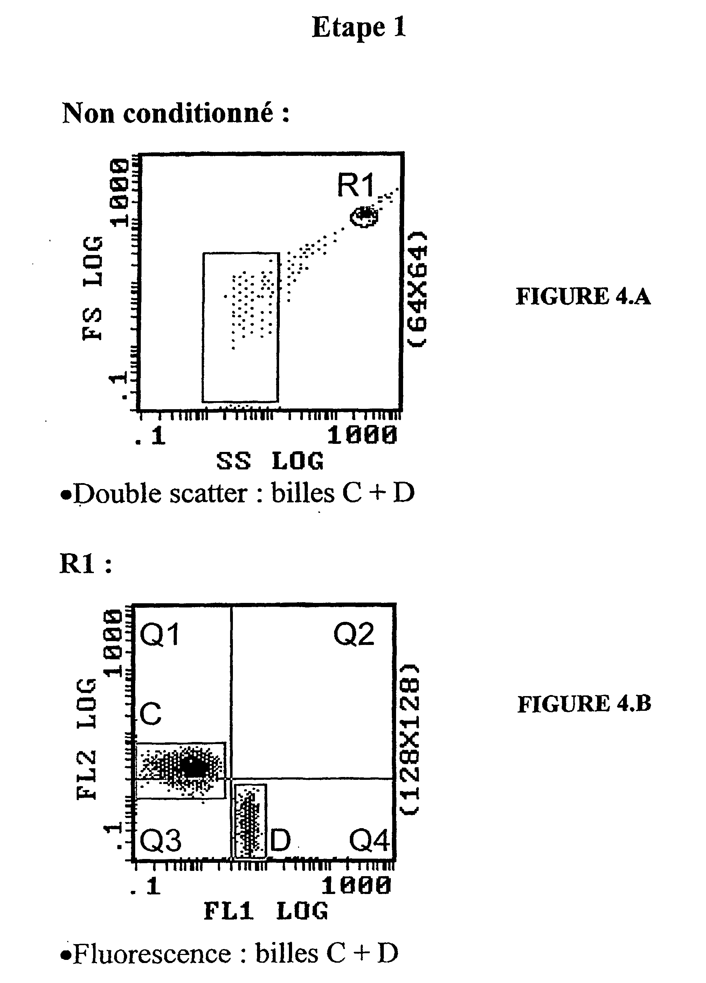

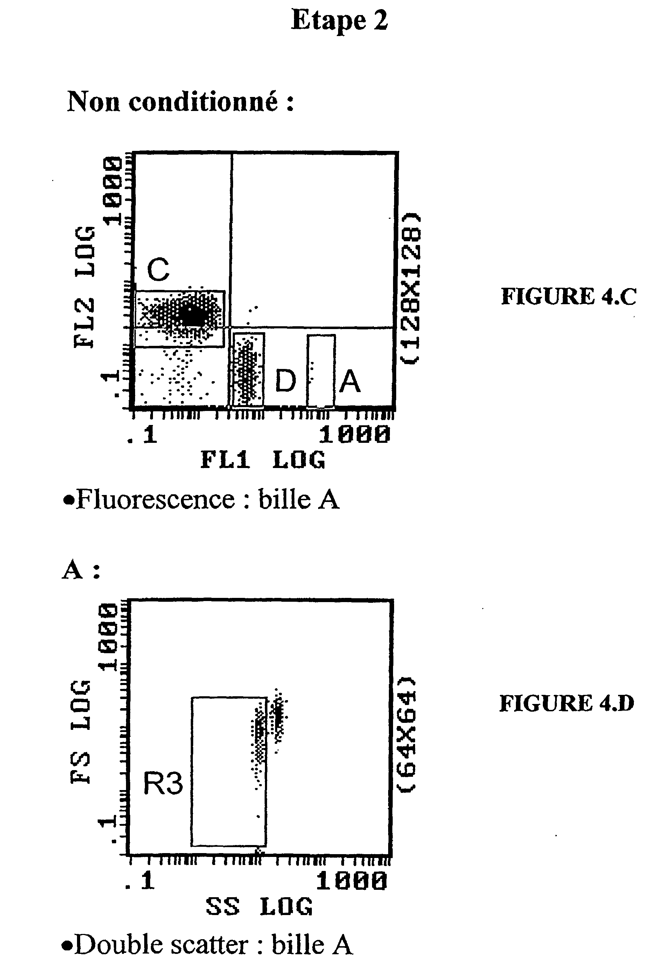

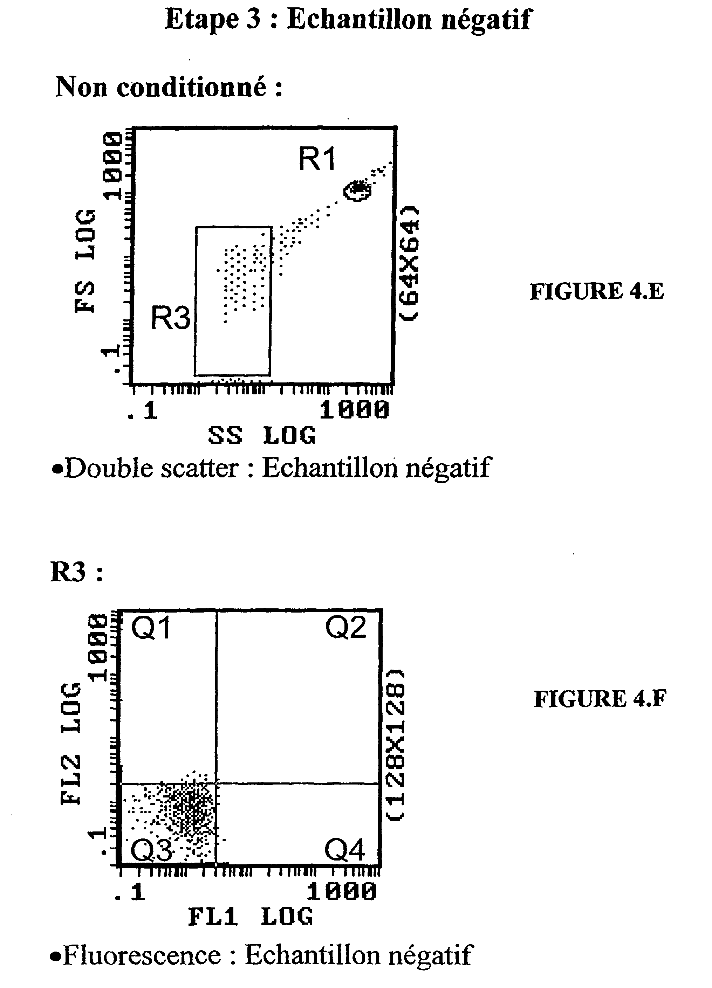

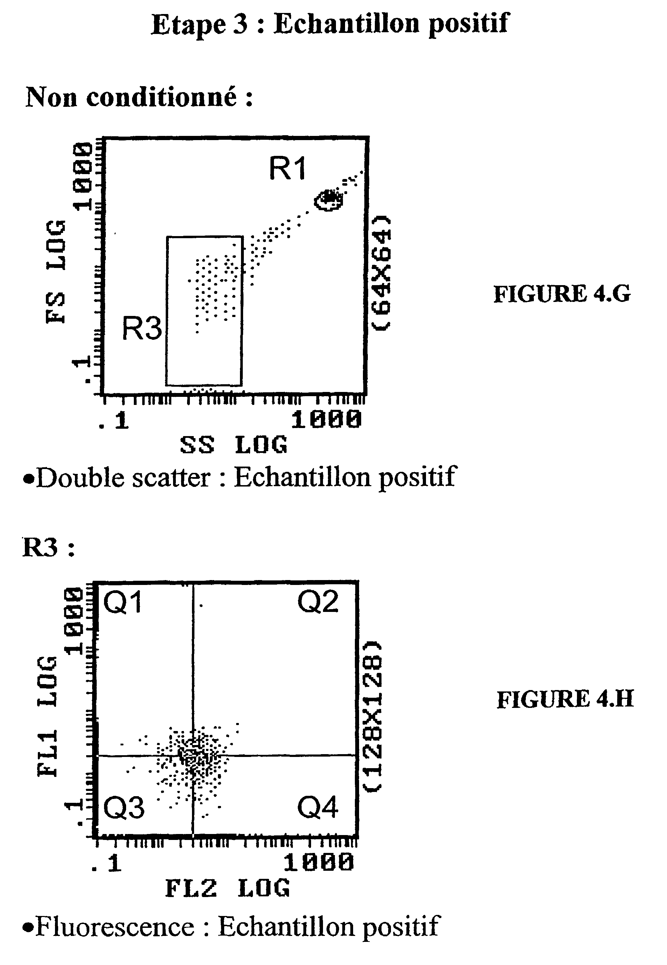

Les cytogrammes présentés aux figures 4.A à 4.H sont une visualisation du test utilisant le monoréactif selon l'invention, suivant les étapes du procédé de l'exemple précédent.
Les expériences ont été réalisées en utilisant un anti-CD51 marqué à la PE (Immunotech) et un anti-CD146 marqué au FITC (Biocytex).
L'échantillon négatif est un plasma normal citraté.
L'échantillon positif est un plasma déplété en plaquettes provenant d'un patient atteint d'un lupus anticoagulant. La lecture cytométique a été réalisée en comptant 20 000 billes étalon interne. Le nombre de microvésicules doublement marquées, localisées dans la région Q2 du cytogramme est déduit du comptage des billes.

**Revendications**

1. Monoréactif pour la détection et la quantification des microparticules d'origine endothéliale dans un échantillon plasmatique par cytométrie de flux, ledit monoréactif comprenant

(i) un mélange d'anticorps monoclonaux spécifiques des molécules membranaires des cellules endothéliales, ledit mélange comprenant un anticorps (1) marqué avec un fluorochrome 1 et un anticorps (2) marqué avec un fluorochrome 2, lesdits anticorps (1) et (2) n'ayant pas la même spécificité, et
(ii) un mélange de microsphères comprenant :

- une population A de microsphères (étalon) marquée par un fluorochrome servant à définir la région d'analyse des microparticules en terme de taille (paramètres de diffusion lumineuse) et permettant leur différenciation,
- une population de microsphères B (étalon), dont le nombre est connu, servant au comptage des microparticules,
- une population de microsphères C marquée uniquement par le fluorochrome 1, servant à définir sur le paramètre de fluorescence du fluorochrome 1 le seuil d'intensité minimum caractérisant les microparticules marquées par les anticorps-fluorochrome 1,
- et une population de microsphères D, de diamètre identique à C, et marquée uniquement par le fluorochrome 2, servant à définir la région d'analyse des événements considérés positifs pour le fluorochrome 2.

2. Monoréactif selon la revendication 1 **caractérisé en ce que** les anticorps sont sélectionnés notamment parmi les anti-CD51, anti-CD34, anti-CD9, et anti-CD146,

3. Monoréactif selon la revendication 2 **caractérisé en ce que** les anticorps sont un anti-CD51 et anti-CD146.

4. Monoréactif selon l'une des revendications 1 à 3 **caractérisé en ce que** les microsphères A ont un diamètre compris entre 0,5 et 1 µm, préférentiellement 0,8 µm, et les microsphères B et C ont un diamètre compris entre 2 et 10 µm, préférentiellement 3 µm.

5. Monoréactif selon l'une des revendications 1 à 4 **caractérisé en ce que** le diamètre des microsphères C et D est différent de celui des billes B de manière à dissocier de façon simple par les paramètres de diffusion lumineuse les populations C + D de la population B.

6. Monoréactif selon l'une des revendications 1 à 5 **caractérisé en ce que** le diamètre des microsphères B, C et D est identique.

7. Monoréactif selon l'une des revendications 1 à 6 **caractérisé en ce que** les populations C et D sont présentes dans une proportion relative de 1/3 à 2/3, préférentiellement 1/2.

8. Monoréactif selon l'une des revendications 1 à 7 **caractérisé en ce que** les fluorochromes sont choisis notamment parmi la phycoérythrine et le FITC.

9. Trousse de diagnostique comprenant un monoréactif selon l'une des revendications 1 à 8 destinée à la détection et à la quantification de microparticules endothéliales dans le plasma humain.

10. Trousse de diagnostique selon la revendication 9 comprenant en outre un échantillon contrôle négatif de référence provenant d'un pool de plasmas humains normaux déplétés en plaquettes, ledit échantillon étant sous la forme liquide ou lyophilisé.

11. Procédé de quantification des microparticules endothéliales dans le sang **caractérisé en ce qu'**il comprend les étapes suivantes :

   a) Préparation de plasma déplété en plaquettes par centrifugation et microfiltration,
   b) Incubation du plasma avec un monoréactif selon l'une des revendications 1 à 8 de sorte à obtenir un marquage en double couleur desdites microparticules,
   c) Discrimination et comptage des microparticules par cytométrie de flux.

12. Procédé selon la revendication 11 **caractérisé en ce que** le plasma déplété en plaquettes est obtenu par double centrifugation et filtration sur Millipore 1μm.

13. Utilisation d'un monoréactif selon l'une des revendications 1 à 8 ou d'une trousse selon l'une des revendications 9 et 10 pour la détection des activités procoagulantes au cours des maladies thrombotiques ou prothrombotiques telles que les lupus anticoagulants.

14. Utilisation d'un monoréactif selon l'une des revendications 1 à 8 ou d'une trousse selon l'une des revendications 9 et 10 pour la détection de lésions vasculaires aiguës ou chroniques.

15. Utilisation d'un monoréactif selon l'une des revendications 1 à 8 pour le marquage en double couleur des microparticules endothéliales de telle sorte que la différenciation entre lesdites microparticules et leur équivalent, notamment leur équivalent plaquettaire, est optimisée.

16. Utilisation d'un monoréactif selon l'une des revendications 1 à 8 pour la standardisation des seuils de discrimination des microparticules endothéliales des autres particules et débris susceptibles d'êtres responsables d'un bruit de fond.

17. Utilisation d'un monoréactif selon l'une des revendications 1 à 8 pour la standardisation du comptage des microparticules endothéliales par rapport aux microsphères étalons.


**Patentansprüche**

1. Monoreagens für den Nachweis und die Quantifizierung von Mikroteilchen endothelialen Ursprungs in einer Plasmaprobe durch Durchflusszytometrie, wobei das sogenannte Monoreagens enthält

   i) eine Mischung von für Membranmoleküle der Endothelzellen spezifischen monoklonalen Antikörpern enthält, wobei die Mischung einen mit einem Fluorochrom 1 markierten Antikörper (1) und einen mit einem Fluorochrom 2 markierten Antikörper (2) umfasst, wobei die Antikörper (1) und (2) nicht dieselbe Spezifität aufweisen, und
   ii) eine Mischung von Mikrokugeln, umfassend

   - eine mit einem Fluorochrom markierte Population A von Mikrokugeln (Eichprobe), die dazu dient, den Analysenbereich der Mikroteilchen in Hinblick auf die Größe (Lichtdiffusionsparameter) zu definieren, und deren Differenzierung erlaubt,
   - eine Population von Mikrokugeln B (Eichprobe), deren Anzahl bekannt ist und die der Zählung der Mikroteilchen dient,
   - eine einzig mit dem Fluorochrom 1 markierte Population von Mikrokugeln C, die dazu dient, hinsichtlich des Fluoreszenzparameters des Fluorochroms 1 den minimalen Intensitätsschwellenwert, der die mit den Fluorochrom-1-Antikörpern markierten Mikroteilchen charakterisiert, zu definieren,
   - und eine Population von Mikrokugeln D mit identischem Durchmesser wie C und einzig mit dem Fluorochrom 2 markiert, die dazu dient, den Analysenbereich der hinsichtlich des Fluorochroms 2 als positiv angesehenen Ereignisse zu definieren.

2. Monoreagens nach Anspruch 1, **dadurch gekennzeichnet, dass** die Antikörper insbesondere unter den anti-CD51, anti-CD34, anti-CD9 und anti-CD146 ausgewählt sind.

3. Monoreagens nach Anspruch 2, **dadurch gekennzeichnet, dass** die Antikörper ein anti-CD51 und anti-CD146 sind.

**4.** Monoreagens nach einem der Ansprüche 1 bis 3, **dadurch gekennzeichnet, dass** die Mikrokugeln A einen Durchmesser zwischen 0,5 und 1 μm, vorzugsweise 0,8 μm haben und die Mikrokugeln B und C einen Durchmesser zwischen 2 und 10 um, vorzugsweise 3 μm haben.

**5.** Monoreagens nach einem der Ansprüche 1 bis 4, **dadurch gekennzeichnet, dass** der Durchmesser der Mikrokugeln C und D sich von jenem der Kugeln B so unterscheidet, dass die Populationen C + D auf einfache Weise anhand der Lichtdiffusionsparameter von der Population B getrennt werden können.

**6.** Monoreagens nach einem der Ansprüche 1 bis 5, **dadurch gekennzeichnet, dass** der Durchmesser der Mikrokugeln B, C und D identisch ist.

**7.** Monoreagens nach einem der Ansprüche 1 bis 6, **dadurch gekennzeichnet, dass** die Populationen C und D in einem relativen Anteil von 1/3 bis 2/3, vorzugsweise 1/2 vorhanden sind.

**8.** Monoreagens nach einem der Ansprüche 1 bis 7, **dadurch gekennzeichnet, dass** die Fluorochrome insbesondere unter Phycoerythrin und FITC ausgewählt sind.

**9.** Diagnostischer Kit, umfassend ein Monoreagens nach einem der Ansprüche 1 bis 8, der für den Nachweis und die Quantifizierung von endothelialen Mikroteilchen in menschlichem Plasma bestimmt ist.

**10.** Diagnostischer Kit nach Anspruch 9, umfassend außerdem eine Negativkontroll-Referenzprobe, die aus einem Pool von normalen plättchenarmen menschlichen Plasmas stammt, wobei die Probe in flüssiger oder lyophilisierter Form vorliegt.

**11.** Verfahren zur Quantifizierung von endothelialen Mikroteilchen im Blut, **dadurch gekennzeichnet, dass** es die folgenden Schritte umfasst:

a) Herstellung von plättchenarmem Plasma durch Zentrifugation und Mikrofiltration, ,
b) Inkubation des Plasmas mit einem Monoreagens nach einem der Ansprüche 1 bis 8 dergestalt, dass eine Doppelfarbmarkierung der Mikroteilchen erhalten wird,
c) Unterscheidung und Zählung der Mikroteilchen durch Durchflusszytometrie.

**12.** Verfahren nach Anspruch 11, **dadurch gekennzeichnet, dass** das plättchenarme Plasma durch doppelte Zentrifugation und Filtration durch Millipore 1 μm erhalten wird.

**13.** Verwendung eines Monoreagens nach einem der Ansprüche 1 bis 8 oder eines Kits nach einem der Ansprüche 9 und 10 für den Nachweis der prokoagulierenden Aktivitäten während thrombotischer oder prothrombotischer Erkrankungen, wie den Lupusantikoagulantien.

**14.** Verwendung eines Monoreagens nach einem der Ansprüche 1 bis 8 oder eines Kits nach einem der Ansprüche 9 und 10 für den Nachweis von akuten oder chronischen Gefäßläsionen.

**15.** Verwendung eines Monoreagens nach einem der Ansprüche 1 bis 8 für die Doppelfarbmarkierung der endothelialen Mikroteilchen dergestalt, dass die Differenzierung zwischen den Mikroteilchen und deren Äquivalent, insbesondere deren Plättchen-Äquivalent, optimiert wird.

**16.** Verwendung eines Monoreagens nach einem der Ansprüche 1 bis 8 für die Standardisierung der Unterscheidungsschwellenwerte der endothelialen Mikroteilchen von den anderen Teilchen und Zelltrümmern, die für ein Hintergrundrauschen verantwortlich sein können.

**17.** Verwendung eines Monoreagens nach einem der Ansprüche 1 bis 8 für die Standardisierung der Zählung der endothelialen Mikroteilchen bezogen auf die Mikrokugeln der Eichproben.

**Claims**

**1.** Monoreagent for detecting and quantifying microparticles of endothelial origin in a plasma sample by flow cytometry, said monoreagent comprising

i) a mixture of monoclonal antibodies specific for endothelial cell membrane molecules, said mixture comprising an antibody (1) labelled with a fluorochrome 1 and an antibody (2) labelled with a fluorochrome 2, said antibodies (1) and (2) not having the same specificity, and
ii) a mixture of microspheres, comprising:

- a population A of microspheres (standard) labelled with a fluorochrome used to define the region of analysis of the microparticles in terms of size (light scattering parameters) and allowing differentiation thereof,
- a population of microspheres B (standard), the number of which is known, used to count the microparticles,
- a population of microspheres C labelled only with fluorochrome 1, used to define, with respect to the fluorochrome 1 fluorescence parameter, the minimum intensity threshold characterizing the microparticles labelled with the fluorochrome-1 antibodies,

- and a population of microspheres D, of diameter identical to C, and labelled only with fluorochrome 2, used to define the region of analysis of the events considered to be positive for fluorochrome 2.

2. Monoreagent according to Claim 1, **characterized in that** the antibodies are selected in particular from anti-CD51, anti-CD34, anti-CD9 and anti-CD146 antibodies.

3. Monoreagent according to Claim 2, **characterized in that** the antibodies are an anti-CD51 and an anti-CD146.

4. Monoreagent according to one of Claims 1 to 3, **characterized in that** the microspheres A are between 0.5 and 1 μm, preferably 0.8 μm, in diameter and the microspheres B and C are between 2 and 10 μm, preferentially 3 μm, in diameter.

5. Monoreagent according to one of Claims 1 to 4, **characterized in that** the diameter of the microspheres C and D is different to that of the beads B so as to dissociate populations C + D from population B simply, by the light scattering parameters.

6. Monoreagent according to one of Claims 1 to 5, **characterized in that** the diameter of the microspheres B, C and D is identical.

7. Monoreagent according to one of Claims 1 to 6, **characterized in that** populations C and D are present in a relative proportion of 1/3 to 2/3, preferentially 1/2.

8. Monoreagent according to one of Claims 1 to 7, **characterized in that** the fluorochromes are chosen in particular from phycoerythrin and FITC.

9. Diagnostic kit comprising a monoreagent according to one of Claims 1 to 8, intended for detecting and quantifying endothelial microparticles in human plasma.

10. Diagnostic kit according to Claim 9, also comprising a reference negative control sample originating from a pool or platelet-depleted normal human plasmas, said sample being in the liquid or lyophilized form.

11. Method for quantifying endothelial microparticles in blood, **characterized in that** it comprises the following steps:

a) preparing platelet-depleted plasma by centrifugation and microfiltration,
b) incubating the plasma with a monoreagent according to one of Claims 1 to 8, so as to obtain double colour labelling of said microparticles,
c) discriminating and counting microparticles by flow cytometry.

12. Method according to Claim 11, **characterized in that** the platelet-depleted plasma is obtained by double centrifugation and filtration through 1 μm Millipore.

13. Use of a monoreagent according to one of Claims 1 to 8, or of a kit according to either of Claims 9 and 10, for detecting procoagulant activities in the course of thrombotic or prothrombotic diseases, such as lupus anticoagulants.

14. Use of a monoreagent according to one of Claims 1 to 8, or of a kit according to either of Claims 9 and 10, for detecting acute or chronic vascular lesions.

15. Use of a monoreagent according to one of Claims 1 to 8, for double colour labelling endothelial microparticles such that differentiation between said microparticles and their equivalent, in particular their platelet equivalent, is optimized.

16. Use of a monoreagent according to one of Claims 1 to 8, for standardizing the thresholds for discrimination between endothelial microparticles and other particles and debris liable to be responsible for background noise.

17. Use of a monoreagent according to one of Claims 1 to 8, for standardizing the counting of endothelial microparticles relative to standard microspheres.

**FIGURE 1**

EP 1 192 443 B1

**FIGURE 2**

EP 1 192 443 B1

EP 1 192 443 B1

R3 :

LFS

LSS

R1

R2

R3

Q1

Q2

Q4

LFL2

LFL1

μP endoth.

μS A

FIGURE 3

# Etape 1

## Non conditionné :

**FIGURE 4.A**

•Double scatter : billes C + D

## R1 :

**FIGURE 4.B**

•Fluorescence : billes C + D

# Etape 2

## Non conditionné :

FIGURE 4.C

•Fluorescence : bille A

## A :

FIGURE 4.D

•Double scatter : bille A

# Etape 3 : Echantillon négatif

## Non conditionné :

FIGURE 4.E

•Double scatter : Echantillon négatif

## R3 :

FIGURE 4.F

•Fluorescence : Echantillon négatif

# Etape 3 : Echantillon positif

## Non conditionné :

FIGURE 4.G

•Double scatter : Echantillon positif

## R3 :

FIGURE 4.H

•Fluorescence : Echantillon positif